# EUROPEAN PATENT SPECIFICATION

(11) **EP 2 596 790 B1**
(45) Date of publication and mention of the grant of the patent: **09.12.2015**
(21) Application number: 13154157.5
(22) Date of filing: 03.03.2009
(51) Int. Cl.: C07D 417/14, A61K 31/4439, A61P 35/00

(54) **Pim kinase inhibitors and methods of their use**
PIM-Kinasehemmer und Verfahren zu ihrer Verwendung
Inhibiteurs de kinase PIM et leurs procédés d'utilisation

(30) Priority: 03.03.2008 US 33359 P
(43) Date of publication of application: 29.05.2013
(62) Divisional of application: 09717066.6
(73) Proprietor: Novartis AG, 4056 Basel (CH)
(72) Inventor: Burger, Matthew, Emeryville, CA California 94608-2916 (US); Lan, Jiong, Emeryville, CA California 94608-2916 (US); Lindvall, Mika, Emeryville, CA California 94608-2916 (US); Nishiguchi, Gisele, Emeryville, CA California 94608-2916 (US); Tetalman, Michelle, Emeryville, CA California 94608-2916 (US)
(74) Representative: Wiessner, Michael

(56) References cited:
- WO-A1-2008/054701
- WO-A1-2008/054702
- WO-A1-2008/054749
- WO-A2-2007/058942

## Description

### FIELD OF THE INVENTION

The present invention relates to new compounds, their tautomers, stereoisomers and polymorphs, and pharmaceutically acceptable salts thereof, compositions of the new compounds together with pharmaceutically acceptable carriers, and uses of the new compounds, either alone or in combination with at least one additional therapeutic agent, in the prophylaxis or treatment of cancer.

### BACKGROUND

Infection with the Maloney retrovirus and genome integration in the host cell genome results in development of lymphomas in mice. Provirus Integration of Maloney Kinase (PIM-Kinase) was identified as one of the frequent proto-oncogenes capable of being transcriptionally activated by this retrovirus integration event (Cuypers HT et al., "Murine leukemia virus-induced T-cell lymphomagenesis: integration of proviruses in a distinct chromosomal region," Cell 37(1):141-50 (1984); Selten G, et al., "Proviral activation of the putative oncogene Pim-1 in MuLV induced T-cell lymphomas" EMBO J 4(7): 1793-8 (1985)), thus establishing a correlation between over-expression of this kinase and its oncogenic potential. Sequence homology analysis demonstrated that there are 3 highly homologous Pim-Kinases (Pim1, 2 & 3), Pim1 being the proto-oncogene originally identified by retrovirus integration. Furthermore, transgenic mice over-expressing Pim1 or Pim2 show increased incidence of T-cell lymphomas (Breuer M et al., "Very high frequency of lymphoma induction by a chemical carcinogen in pim-1 transgenic mice" Nature 340(6228):61-3 (1989)), while over-expression in conjunction with c-myc is associated with incidence of B-cell lymphomas (Verbeek S et al., "Mice bearing the E mu-myc and E mu-pim-1 transgenes develop pre-B-cell leukemia prenatally" Mol Cell Biol 11(2):1176-9 (1991)). Thus, these animal models establish a strong correlation between Pim over-expression and oncogenesis in hematopoietic malignancies. In addition to these animal models, Pim over-expression has been reported in many other human malignancies. Pim1, 2 & 3 over-expression is frequently observed in many hematopoietic maligmancies (Amson R et al., "The human protooncogene product p33pim is expressed during fetal hematopoiesis and in diverse leukemias," PNAS USA 86(22):8857-61 (1989); Cohen AM et al., "Increased expression of the hPim-2 gene in human chronic lymphocytic leukemia and non-Hodgkin lymphoma," Leuk Lymph 45(5):951-5 (2004), Huttmann A et al., "Gene expression signatures separate B-cell chronic lymphocytic leukaemia prognostic subgroups defined by ZAP-70 and CD38 expression status," Leukemia 20:1774-1782 (2006)) and in prostate cancer (Dhanasekaran SM, et al., "Delineation of prognostic biomarkers in prostate cancer," Nature 412(6849):822-6 (2001); Cibull TL, et al., "Overexpression of Pim-1 during progression of prostatic adenocarcinoma," J Clin Pathol 59(3):285-8 (2006)), while over-expression of Pim3 is frequently observed in hepatocellular carcinoma (Fujii C, et al., "Aberrant expression of serine/threonine kinase Pim-3 in hepatocellular carcinoma development and its role in the proliferation of human hepatoma cell lines," Int J Cancer 114:209-218 (2005)) and pancreatic cancer (Li YY et al., "Pim-3, a proto-oncogene with serine/threonine kinase activity, is aberrantly expressed in human pancreatic cancer and phosphorylates bad to block bad-mediated apoptosis in human pancreatic cancer cell lines," Cancer Res 66(13):6741-7 (2006)).

Pim1, 2 & 3 are Serine/Threonine kinases normally function in survival and proliferation of hematopoietic cells in response to growth factors and cytokines. Cytokines signaling through the Jak/Stat pathway leads to activation of transcription of the Pim genes and synthesis of the proteins. No further post-translational modifications are required for the Kinase Pim activity. Thus, signaling down stream is primarily controlled at the transcriptional/translational and protein turnover level. Substrates for Pim kinases include regulators of apoptosis such as the Bcl-2 family member BAD (Aho T et al., "Pim-1 kinase promotes inactivation of the pro-apoptotic Bad protein by phosphorylating it on the Ser112 gatekeeper site,: FEBS Letters 571: 43-49 (2004)), cell cycle regulators such as p21^{WFA1/CIP1} (Wang Z, et al., "Phosphorylation of the cell cycle inhibitor p21Cip1/WAF1 by Pim-1 kinase," Biochim Biophys Acta 1593:45- 55 (2002)), CDC25A (1999), C-TAK (Bachmann M et al., "The Oncogenic Serine/Threonine Kinase Pim-1 Phosphorylates and Inhibits the Activity of Cdc25C-associated Kinase 1 (C-TAK1). A novel role for Pim-1 at the G2/M cell cycle checkpoint," J Biol Chem 179:48319-48328 (2004)) and NuMA (Bhattacharya N, et al., "Pim-1 associates with protein complexes necessary for mitosis, "Chromosoma 111(2):80-95 (2002)) and the protein synthesis regulator 4EBP1 (Hammerman PS et al, "Pim and Akt oncogenes are independent regulators of hematopoietic cell growth and survival," Blood 105(11):4477-83 (2005)). The effects of Pim(s) in these regulators are consistent with a role in protection from apoptosis and promotion of cell proliferation and growth. Thus, over-expression of Pim(s) in cancer is thought to play a role in promoting survival and proliferation of cancer cells and, therefore, their inhibitions should be an effective way of treating cancers on which they are over-expressed. In fact several reports indicate that knocking down expression of Pim(s) with siRNA results in inhibition of proliferation and cell death (Dai JM, et al., "Antisense oligodeoxynucleotides targeting the serine/threonine kinase Pim-2 inhibited proliferation of DU-145 cells," Acta Pharmacol Sin 26(3):364-8 (2005); Fujii et al 2005; Li et al 2006). Furthermore, mutational activation of several well know oncogenes in hematopoietic malignancies are thought exert its effects at least in part through Pim(s). For example, targeted down regulation of pim expression impairs survival of hematopoietic cells transformed by Flt3 and BCR/ABL (Adam et al 2006). Thus, inhibitors to Pim1, 2 &3 would be useful in the treatment of these malignancies. In addition to a potential role in cancer treatment and myeloproliferative diseases, such inhibitor could be useful to control expansion of immune cells in other pathologic condition such as autoimmune diseases, allergic reactions and in organ transplantation rejection syndromes. This notion is supported by the findings that differentiation of Th1 Helper T-cells by IL-12 and IFN-α results in induction of expression of both Pim1&2 (Aho T et al, "Expression of human Pim family genes is selectively up-regulated by cytokines promoting T helper type 1, but not T helper type 2, cell differentiation," Immunology 116: 82-88 (2005)). Moreover, Pim(s) expression is inhibited in both cell types by the immunosuppressive TGF-β (Aho et al 2005). These results suggest that Pim kinases are involved in the early differentiation process of Helper T-cells, which coordinate the immunological responses in autoimmune diseases, allergic reaction and tissue transplant rejection.

WO08/054749, WO08/054701 and WO08/054702 disclose anilinopiperazine derivatives as protein kinase inhibitors. A continuing need exists for compounds that inhibit the proliferation of capillaries, inhibit the growth of tumors, treat cancer, modulate cell cycle arrest, and/or inhibit molecules such as Pim1, Pim2, and Pim3 and pharmaceutical formulations and medicaments that contain such compounds. A need also exists for methods of administering such compounds, pharmaceutical formulations, and medicaments to patients or subjects in need thereof.

### SUMMARY OF THE INVENTION

The present invention provides a compound of Formula I, or a stereoisomer, tautomer, or pharmaceutically acceptable salt thereof, 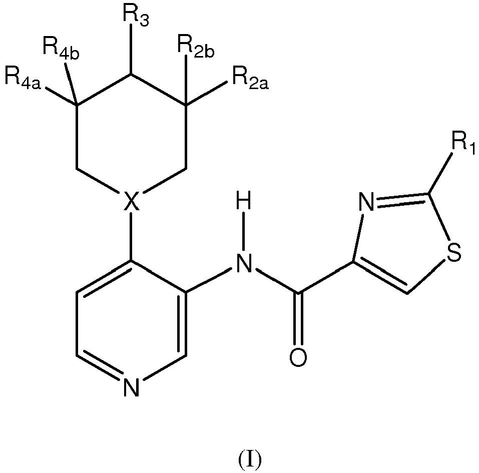 wherein,
R₁ is selected from 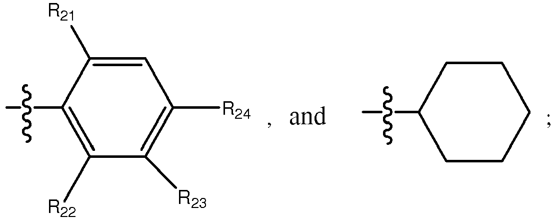
X represents CH, or N;
R₂ₐ is selected from amino, methyl, CH₂F, CF₃, C₂H₅, and H;
R_{2b} is selected from H, and methyl;
R₃ is selected from H, OH, OCH₃, CH₃, F, and Cl;
R₄ₐ is selected from , OH, OCH₃, OC₂H₅ and F;
R_{4b} is selected from methyl, H, and F;
R₂₁ represents H or F;
R₂₂ represents H, Cl, or F;
R₂₃ represents F, OC₂H₅, OCH₃, Cl, H, methyl, OH, or OCH(CH₃)₂; and
R₂₄ represents H or OH.

Another aspect of the present invention provides a composition comprising a therapeutically effective amount of compound of Formula I, or a stereoisomer, tautomer, or pharmaceutically acceptable salt thereof, together with a pharmaceutically acceptable carrier.

Provided in another aspect of the present invention is a method for inhibiting PIM kinase activity in a cell, comprising contacting the cell with an effective amount of a compound of Formula I. Yet another aspect of the present invention provides a method for treating a condition by modulation of Provirus Integration of Maloney Kinase (PIM kinase) activity comprising administering to a patient in need of such treatment an effective amount of a compound of Formula I.

### DETAILED DESCRIPTION OF THE INVENTION

In one aspect, the present invention provides compounds of Formula I, or a stereoisomer, tautomer, or pharmaceutically acceptable salt thereof, 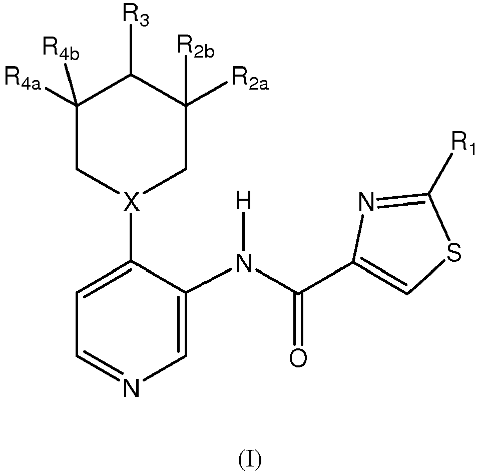 wherein,
R₁ is selected from 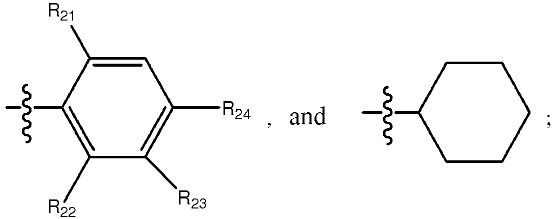
X represents CH, or N;
R₂ₐ is selected from amino, methyl, CH₂F, CF₃, C₂H₅, and H;
R_{2b} is selected from H, and methyl;
R₃ is selected from H, OH, OCH₃, CH₃, F, and Cl;
R₄ₐ is selected from OH, OCH₃, OC₂H₅ and F;
R_{4b} is selected from methyl, H, and F;
R₂₁ represents H or F;
R₂₂ represents H, Cl, or F;
R₂₃ represents F, OC₂H₅, OCH₃, Cl, H, methyl, OH, or OCH(CH₃)₂; and
R₂₄ represents H or OH.

Another aspect of the present invention provides a composition comprising a therapeutically effective amount of compound of Formula I, or a stereoisomer, tautomer, or pharmaceutically acceptable salt thereof, together with a pharmaceutically acceptable carrier.

Provided in another aspect of the present invention is a method for inhibiting PIM kinase activity in a cell, comprising contacting the cell with an effective amount of a compound of Formula I. Yet another aspect of the present invention provides a method for treating a condition by modulation of Provirus Integration of Maloney Kinase (PIM kinase) activity comprising administering to a patient in need of such treatment an effective amount of a compound of Formula I.

A preferred embodiment of this aspect of the present invention provides a method for treating a cancer disorder in a patient, comprising administering to the patient a composition comprising an amount of a compound of Claim 1 or Claim 10 effective to inhibit PIM kinase activity in the patient

Other aspect of the present invention provides a compound of any Formula Ifor use as a therapeutic agent. Yet another aspect of the present invention provides the use of any one of the compounds of Formula I in the manufacture of a medicament for the treatment of cancer.

### Definitions

"PIM inhibitor" is used herein to refer to a compound that exhibits an IC₅₀ with respect to PIM Kinase activity of no more than about 100 µM and more typically not more than about 50 µM, as measured in the PIM depletion assays described herein below.

The phrase "alkyl" refers to alkyl groups that do not contain heteroatoms. Thus the phrase includes straight chain alkyl groups such as methyl, ethyl, propyl, butyl, pentyl, hexyl, heptyl, octyl, nonyl, decyl, undecyl, dodecyl and the like.

As used herein, the term "halogen" or "halo" refers to chloro, bromo, fluoro and iodo groups. "Haloalkyl" refers to an alkyl radical substituted with one or more halogen atoms. The term "haloloweralkyl" refers to a lower alkyl radical substituted with one or more halogen atoms. The term "haloalkoxy" refers to an alkoxy radical substituted with one or more halogen atoms. The term "haloloweralkoxy" refers to a loweralkoxy radical substituted with one or more halogen atoms.

"Amino" refers herein to the group -NH₂, which may be substituted to form -NRR'. The term "alkylamino" refers herein to the group -NRR' where R and R' are each independently selected from hydrogen or a lower alkyl. The term "arylamino" refers herein to the group -NRR' where R is aryl and R' is hydrogen, a lower alkyl, or an aryl. The term "aralkylamino" refers herein to the group -NRR' where R is a lower aralkyl and R' is hydrogen, a loweralkyl, an aryl, or a loweraralkyl.

The term "alkoxy" refers to RO- wherein R is substituted or unsubstituted alkyl. Representative examples of loweralkoxy groups include methoxy, ethoxy, *t*-butoxy, trifluoromethoxy and the like.

"Cycloalkyl" refers to a mono- or polycyclic, heterocyclic or carbocyclic alkyl substituent. Typical cycloalkyl substituents have from 3 to 8 backbone (i.e., ring) atoms in which each backbone atom is either carbon or a heteroatom. The term "heterocycloalkyl" or "heterocyclyl" refers herein to cycloalkyl substituents that have from 1 to 5, and more typically from 1 to 4 heteroatoms in the ring structure. Suitable heteroatoms employed in compounds of the present invention are nitrogen, oxygen, and sulfur. Representative heterocycloalkyl moieties include, for example, morpholino, piperazinyl, piperidinyl and the like. Carbocycloalkyl groups are cycloalkyl groups in which all ring atoms are carbon. When used in connection with cycloalkyl substituents, the term "polycyclic" refers herein to fused and non-fused alkyl cyclic structures.

"Aryl" refers to optionally substituted monocyclic and polycyclic aromatic groups having from 3 to 14 backbone carbon or hetero atoms, and includes both carbocyclic aryl groups and heterocyclic aryl groups. Carbocyclic aryl groups are aryl groups in which all ring atoms in the aromatic ring are carbon. The term "heteroaryl" refers herein to aryl groups having from 1 to 4 heteroatoms as ring atoms in an aromatic ring with the remainder of the ring atoms being carbon atoms. When used in connection with aryl substituents, the term "polycyclic aryl" refers herein to fused and non-fused cyclic structures in which at least one cyclic structure is aromatic, such as, for example, benzodioxozolo (which has a heterocyclic structure fused to a phenyl group, i.e., 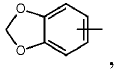 naphthyl, and the like. Exemplary aryl moieties employed as substituents in compounds of the present invention include phenyl, pyridyl, pyrimidinyl, thiazolyl, indolyl, imidazolyl, oxadiazolyl, tetrazolyl, pyrazinyl, triazolyl, thiophenyl, furanyl, quinolinyl, purinyl, naphthyl, benzothiazolyl, benzopyridyl, and benzimidazolyl, and the like.

"Optionally substituted" or "substituted" refers to the replacement of one or more hydrogen atoms with a monovalent or divalent radical. Suitable substitution groups include, for example, hydroxy, nitro, amino, imino, cyano, halo, thio, sulfonyl, thioamido, amidino, imidino, oxo, oxamidino, methoxamidino, imidino, guanidino, sulfonamido, carboxyl, formyl, loweralkyl, haloloweralkyl, loweralkylamino, haloloweralkylamino, loweralkoxy, haloloweralkoxy, loweralkoxyalkyl, alkylcarbonyl, aminocarbonyl, arylcarbonyl, aralkylcarbonyl, heteroarylcarbonyl, heteroaralkylcarbonyl, alkylthio, aminoalkyl, cyanoalkyl, aryl and the like.

The substitution group can itself be substituted. The group substituted onto the substitution group can be carboxyl, halo; nitro, amino, cyano, hydroxy, loweralkyl, loweralkoxy, aminocarbonyl, -SR, thioamido, -SO₃H, -SO₂R or cycloalkyl, where R is typically hydrogen, hydroxyl or loweralkyl.

It is understood that the above definitions are not intended to include impermissible substitution patterns (e.g., methyl substituted with five fluoro groups or a halogen atom substituted with another halogen atom). Such impermissible substitution patterns are well known to the skilled artisan.

It will also be apparent to those skilled in the art that the compounds of the invention, including the compounds of compounds of formula (I) or their stereoisomers, as well as the pharmaceutically acceptable salts, may be subject to tautomerization and may therefore exist in various tautomeric forms wherein a proton of one atom of a molecule shifts to another atom and the chemical bonds between the atoms of the molecules are consequently rearranged. See, e.g., March, Advanced Organic Chemistry: Redactions, Mechanisms and Structures, Fourth Edition, John Wiley & Sons, pages 69-74 (1992). As used herein, the term "tautomer" refers to the compounds produced by the proton shift, and it should be understood that the all tautomeric forms, insofar as they may exist, are included within the invention.

The compounds of the invention, including the compounds of formula (I) or their tautomers, as well as the pharmaceutically acceptable salts, may comprise asymmetrically substituted carbon atoms. Such asymmetrically substituted carbon atoms can result in the compounds of the invention existing in enantiomers, diastereomers, and other stereoisomeric forms that may be defined, in terms of absolute stereochemistry, such as in (R)- or (S)- forms. As a result, all such possible isomers, individual stereoisomers in their optically pure forms, mixtures thereof, racemic mixtures (or "racemates"), mixtures of diastereomers, as well as single diastereomers of the compounds of the invention are included in the present invention. The terms "S" and "R" configuration, as used herein, are as defined by the IUPAC 1974 RECOMMENDATIONS FOR SECTION E, FUNDAMENTAL STEREOCHEMISTRY, Pure Appl. Chem. 45:13-30 (1976). The terms α and β are employed for ring positions of cyclic compounds. The α-side of the reference plane is that side on which the preferred substituent lies at the lower numbered position. Those substituents lying on the opposite side of the reference plane are assigned β descriptor. It should be noted that this usage differs from that for cyclic stereoparents, in which "α" means "below the plane" and denotes absolute configuration. The terms α and β configuration, as used herein, are as defined by the CHEMICAL ABSTRACTS INDEX GUIDE-APPENDIX IV (1987) paragraph 203.

As used herein, the term "pharmaceutically acceptable salts" refers to the nontoxic acid or alkaline earth metal salts of the compounds of Formula (I). These salts can be prepared *in situ* during the final isolation and purification of the compounds of Formula (I), or by separately reacting the base or acid functions with a suitable organic or inorganic acid or base, respectively. Representative salts include but are not limited to the following: acetate, adipate, alginate, citrate, aspartate, benzoate, benzenesulfonate, bisulfate, butyrate, camphorate, camphorsulfonate, digluconate, cyclopentanepropionate, dodecylsulfate, ethanesulfonate, glucoheptanoate, glycerophosphate, hemisulfate, heptanoate, hexanoate, fumarate, hydrochloride, hydrobromide, hydroiodide, 2-hydroxyethanesulfonate, lactate, maleate, methanesulfonate, nicotinate, 2-naphthalenesulfonate, oxalate, pamoate, pectinate, persulfate, 3-phenylproionate, picrate, pivalate, propionate, succinate, sulfate, tartrate, thiocyanate, p-toluenesulfonate and undecanoate. Also, the basic nitrogen-containing groups can be quaternized with such agents as loweralkyl halides, such as methyl, ethyl, propyl, and butyl chloride, bromides, and iodides; dialkyl sulfates like dimethyl, diethyl, dibutyl, and diamyl sulfates, long chain halides such as decyl, lauryl, myristyl and stearyl chlorides, bromides and iodides, aralkyl halides like benzyl and phenethyl bromides, and others. Water or oil-soluble or dispersible products are thereby obtained.

Examples of acids which may be employed to form pharmaceutically acceptable acid addition salts include such inorganic acids as hydrochloric acid, sulfuric acid and phosphoric acid and such organic acids as oxalic acid, maleic acid, methanesulfonic acid, succinic acid and citric acid. Basic addition salts can be prepared *in situ* during the final isolation and purification of the compounds of formula (I), or separately by reacting carboxylic acid moieties with a suitable base such as the hydroxide, carbonate or bicarbonate of a pharmaceutically acceptable metal cation or with ammonia, or an organic primary, secondary or tertiary amine. Pharmaceutically acceptable salts include, but are not limited to, cations based on the alkali and alkaline earth metals, such as sodium, lithium, potassium, calcium, magnesium, aluminum salts and the like, as well as nontoxic ammonium, quaternary ammonium, and amine cations, including, but not limited to ammonium, tetramethylammonium, tetraethylammonium, methylamine, dimethylamine, trimethylamine, triethylamine, ethylamine, and the like. Other representative organic amines useful for the formation of base addition salts include diethylamine, ethylenediamine, ethanolamine, diethanolamine, piperazine and the like.

As used herein, the term "pharmaceutically acceptable ester" refers to esters, which hydrolyze *in vivo* and include those that break down readily in the human body to leave the parent compound or a salt thereof. Suitable ester groups include, for example, those derived from pharmaceutically acceptable aliphatic carboxylic acids, particularly alkanoic, alkenoic, cycloalkanoic and alkanedioic acids, in which each alkyl or alkenyl moiety advantageously has not more than 6 carbon atoms. Examples of particular esters include formates, acetates, propionates, butyrates, acrylates and ethylsuccinates.

The term "pharmaceutically acceptable prodrugs" as used herein refers to those prodrugs of the compounds of the present invention which are, within the scope of sound medical judgment, suitable for use in contact with the tissues of humans and lower animals without undue toxicity, irritation, allergic response, and the like, commensurate with a reasonable benefit/risk ratio, and effective for their intended use, as well as the zwitterionic forms, where possible, of the compounds of the invention. The term "prodrug" refers to compounds that are rapidly transformed *in vivo* to yield the parent compound of the above formula, for example by hydrolysis in blood. A thorough discussion is provided in T. Higuchi and V. Stella, Pro-drugs as Novel Delivery Systems, Vol. 14 of the A.C.S. Symposium Series, and in Edward B. Roche, ed., Bioreversible Carriers in Drug Design, American Pharmaceutical Association and Pergamon Press, 1987.

It will be apparent to those skilled in the art that the compounds of the invention, including the compounds of formula (I) or their tautomers, as well as the pharmaceutically acceptable salts, may be processed *in vivo* through metabolism in a human or animal body or cell to produce metabolites. The term "metabolite" as used herein refers to the formula of any derivative produced in a subject after administration of a parent compound. The derivatives may be produced from the parent compound by various biochemical transformations in the subject such as, for example, oxidation, reduction, hydrolysis, or conjugation and include, for example, oxides and demethylated derivatives. The metabolites of a compound of the invention may be identified using routine techniques known in the art. See, e.g., Bertolini, G. et al., J. Med. Chem. 40:2011-2016 (1997); Shan, D. et al., J. Pharm. Sci. 86(7):765-767; Bagshawe K., Drug Dev. Res. 34:220-230 (1995); Bodor, N., Advances in Drug Res. 13:224-331 (1984); Bundgaard, H., Design of Prodrugs (Elsevier Press 1985); and Larsen, I. K., Design and Application of Prodrugs, Drug Design and Development (Krogsgaard-Larsen et al., eds., Harwood Academic Publishers, 1991[**0039**] The term "cancer" refers to cancer diseases that can be beneficially treated by the inhibition of Pim kinase, including, for example, solid cancers, such as carcinomas (e.g., of the lungs, pancreas, thyroid, ovarian, bladder, breast, prostate, or colon), melanomas, myeloid disorders (e.g., myeloid leukemia, multiple myeloma and erythroleukemia), adenomas (e.g., villous colon adenoma) and sarcomas (e.g., osteosarcoma).

### Synthetic Methods

The compounds of the invention can be obtained through procedures known to the skilled in the art. For example, as shown in Scheme 1, 4-chloro, 3-nitro pyridine can be reacted with a nucleophile yielding after nitro reduction a 4-substituted 3-amino pyridine I. The substituted amino pyridines I can be acylated with thiazolecarboxylic acids with the aid of coupling agents, or with acid halides or acid anhydrides yielding 3, 4 disubstituted pyridines II. If the 2 position R group of the thiazole is bromo, triflate or iodo, further modification to incorporate a variety of substituents at this position can be realized by metal mediated carbon-carbon bond forming reactions.

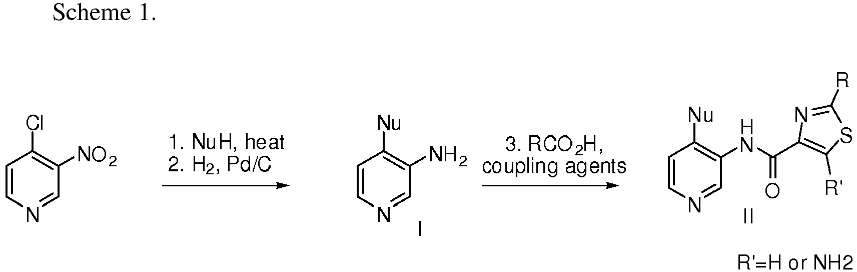

The reaction of 4-chloro-3-nitropyridine with nucleophiles as depicted in Scheme 1 is not limited to nitrogen based nucleophiles; carbon-carbon bonds can be formed as well with the net addition of carbon nucleophiles. As shown in Scheme 2, cyclohexanediones can be converted via monotriflates to the corresponding cyclohexenoneboronate esters which can undergo palladium mediated carbon-carbon bond formation with 4-chloro, 3-nitro pyridine to yield nitropyridine substituted cyclohexenones III. Reduction of the enone functionality can yield a cyclohexenol IV which upon alcohol protection, nitro and alkene reduction, amide coupling and deprotection can yield cyclohexanol amides V. Cyclohexenol IV can also undergo Mitsunobo reaction with phthalimide to yield a protected aminocyclohexene V. Following nitro and alkene reduction, phthalimide protected aminocyclohexyl pyridyl aniline VIIa can undergo amide coupling and deprotection, to yield aminocyclohexane amides VIII. The corresponding Boc protected aminocyclohexane pyridyl aniline VIIb can also be prepared from cyclohexenol IV in the following manner: alcohol protection, alkene and nitro reduction, pyridyl amine Cbz protection, silyl ether deprotection, dess-martin oxidation to the cyclohexanone, reductive amination with benzylamine, Cbz and benzyl deprotection and primary aliphatic amine Boc protection. In the amide products V and VIII, if R2 is halo or triflate, the amides IV and VIII can be further modified by standard modifications to introduce substituted aryls, alkyls and heteroaryls at R2. For example, if R2 is Br, by reaction with boronic acids or organometallic reagents, or conversion to the corresponding boronate ester and reaction with aryl/heteroaryl halides or triflates, a variety of R2 modifications are possible.

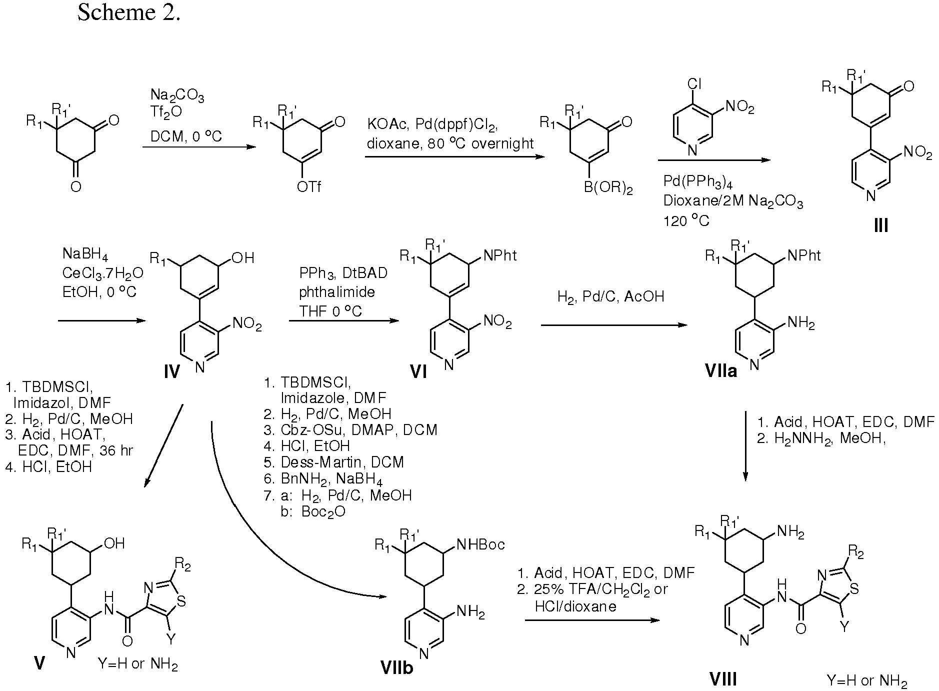

Thiazole amides with substituted cyclohexyl groups can be obtained by modification of nitropyridyl cyclohexenol IV. As shown in Scheme 3, cyclohexenol IV can be dehydrated yielding a cyclohexadiene which upon epoxidation (via bromohydrin formation and HBr elimination or from mCPBA directly) and azide epoxide opening yields cyclohexenyl azido alcohol IX. Cyclohexenyl azido alcohol IX can be converted to the trans protected amino hydroxy aniline Xa by azide reduction, alcohol protection and alkene and nitro reduction. Alternatively, the cyclohexenyl azido alcohol IX can be converted to the protected *cis* amino hydroxy aniline Xb by azide reduction and Boc protection, alcohol mesylation and intramolecular cyclization to the cis cyclic carbamate, followed by Boc protection and alkene and nitro reduction. The resulting cyclohexylpyridyl anilines Xa and Xb can be converted to the corresponding thiazole amides XIa and XIb by amide coupling, acetate or cyclic carbamate cleavage and Boc deprotection. If R₂ is halo or triflate, the amides XIa and XIb and XII can be further modified by standard modifications to introduce substituted aryls, alkyls and heteroaryls at R₂ after amide bond formation and prior to full deprotection. For example, if R₂ is Br, by reaction with boronic acids or organometallic reagents, or conversion to the corresponding boronate ester and reaction with aryl/heteroaryl halides or triflates, a variety of R₂ modifications are possible.

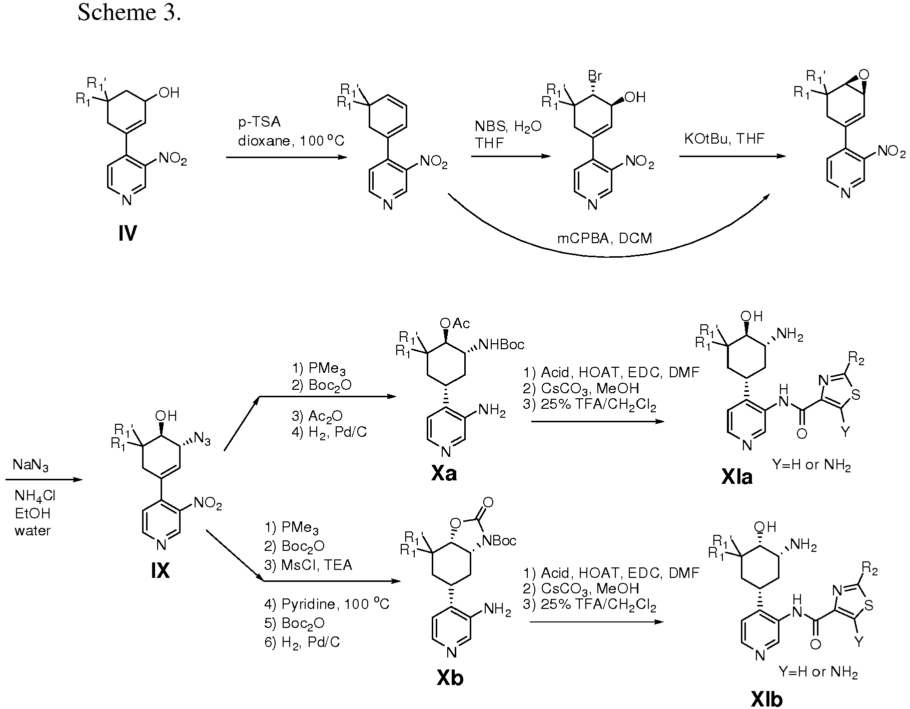

Substitued 3-aminopiperidines can be prepared and modified to yield substituted 3-aminopiperidinyl thiazole amides XII as depicted in Scheme 4.. Reaction of crotyl boronate esters with SerOBn aldehyde followed by cyclic carbamate formation, alkene oxidative cleavage and reduction yields hydroxyl compound XIII. Benzyl deprotection followed by bistosylation and reaction with p-methoxybenzylamine, and amine deprotection yields piperidine XIV. By use of chiral boronate esters, and different L and D serine derived aldehydes, all possible diastereomers of the resulting trisubstituted 5-alkyl, 4-hydroxy, 3-aminopiperidine can be obtained. Reaction of substituted piperidine XIV with 4-chloro-3-nitropyridine, followed by carbamate protection, nitro reduction, amide coupling, cyclic carbamate opening and deprotection yields trisubstituted 5-methyl, 4-hydroxy, 3-aminopiperidinyl thiazole amides XII. If R₂ is halo or triflate, the amide XII can be further modified by standard modifications to introduce substituted aryls, alkyls and heteroaryls at R₂ after amide bond formation and prior to full deprotection. For example, if R₂ is Br, by reaction with boronic acids or organometallic reagents, or conversion to the corresponding boronate ester and reaction with aryl/heteroaryl halides or triflates, a variety of R2 modifications are possible.

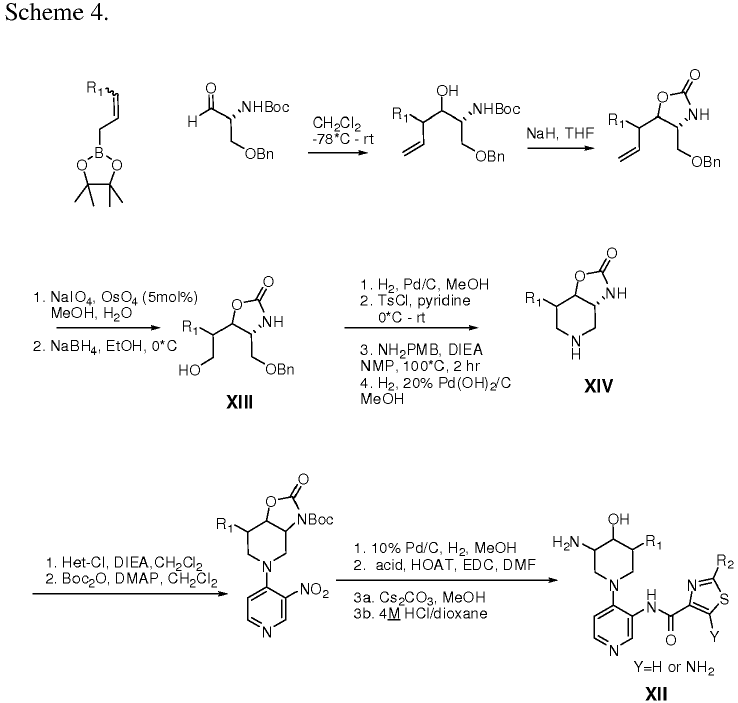

The compounds of the invention are useful *in vitro* or *in vivo* in inhibiting the growth of cancer cells. The compounds may be used alone or in compositions together with a pharmaceutically acceptable carrier or excipient. Suitable pharmaceutically acceptable carriers or excipients include, for example, processing agents and drug delivery modifiers and enhancers, such as, for example, calcium phosphate, magnesium stearate, talc, monosaccharides, disaccharides, starch, gelatin, cellulose, methyl cellulose, sodium carboxymethyl cellulose, dextrose, hydroxypropyl-β-cyclodextrin, polyvinylpyrrolidinone, low melting waxes, ion exchange resins, and the like, as well as combinations of any two or more thereof. Other suitable pharmaceutically acceptable excipients are described in "Remington's Pharmaceutical Sciences," Mack Pub. Co., New Jersey (1991).

Effective amounts of the compounds of the invention generally include any amount sufficient to detectably inhibit Pim activity by any of the assays described herein, by other Pim kinase activity assays known to those having ordinary skill in the art or by detecting an inhibition or alleviation of symptoms of cancer.

The amount of active ingredient that may be combined with the carrier materials to produce a single dosage form will vary depending upon the host treated and the particular mode of administration. It will be understood, however, that the specific dose level for any particular patient will depend upon a variety of factors including the activity of the specific compound employed, the age, body weight, general health, sex, diet, time of administration, route of administration, rate of excretion, drug combination, and the severity of the particular disease undergoing therapy. The therapeutically effective amount for a given situation can be readily determined by routine experimentation and is within the skill and judgment of the ordinary clinician.

For purposes of the present invention, a therapeutically effective dose will generally be a total daily dose administered to a host in single or divided doses may be in amounts, for example, of from 0.001 to 1000 mg/kg body weight daily and more preferred from 1.0 to 30 mg/kg body weight daily. Dosage unit compositions may contain such amounts of submultiples thereof to make up the daily dose.

The compounds of the present invention may be administered orally, parenterally, sublingually, by aerosolization or inhalation spray, rectally, or topically in dosage unit formulations containing conventional nontoxic pharmaceutically acceptable carriers, adjuvants, and vehicles as desired. Topical administration may also involve the use of transdermal administration such as transdermal patches or ionophoresis devices. The term parenteral as used herein includes subcutaneous injections, intravenous, intramuscular, intrasternal injection, or infusion techniques.

Injectable preparations, for example, sterile injectable aqueous or oleaginous suspensions may be formulated according to the known art using suitable dispersing or wetting agents and suspending agents. The sterile injectable preparation may also be a sterile injectable solution or suspension in a nontoxic parenterally acceptable diluent or solvent, for example, as a solution in 1,3-propanediol. Among the acceptable vehicles and solvents that may be employed are water, Ringer's solution, and isotonic sodium chloride solution. In addition, sterile, fixed oils are conventionally employed as a solvent or suspending medium. For this purpose any bland fixed oil may be employed including synthetic mono- or di-glycerides. In addition, fatty acids such as oleic acid find use in the preparation of injectables.

Suppositories for rectal administration of the drug can be prepared by mixing the drug with a suitable nonirritating excipient such as cocoa butter and polyethylene glycols, which are solid at ordinary temperatures but liquid at the rectal temperature and will therefore melt in the rectum and release the drug.

Solid dosage forms for oral administration may include capsules, tablets, pills, powders, and granules. In such solid dosage forms, the active compound may be admixed with at least one inert diluent such as sucrose lactose or starch. Such dosage forms may also comprise, as is normal practice, additional substances other than inert diluents, e.g., lubricating agents such as magnesium stearate. In the case of capsules, tablets, and pills, the dosage forms may also comprise buffering agents. Tablets and pills can additionally be prepared with enteric coatings.

Liquid dosage forms for oral administration may include pharmaceutically acceptable emulsions, solutions, suspensions, syrups, and elixirs containing inert diluents commonly used in the art, such as water. Such compositions may also comprise adjuvants, such as wetting agents, emulsifying and suspending agents, cyclodextrins, and sweetening, flavoring, and perfuming agents.

The compounds of the present invention can also be administered in the form of liposomes. As is known in the art, liposomes are generally derived from phospholipids or other lipid substances. Liposomes are formed by mono- or multilamellar hydrated liquid crystals that are dispersed in an aqueous medium. Any nontoxic, physiologically acceptable and metabolizable lipid capable of forming liposomes can be used. The present compositions in liposome form can contain, in addition to a compound of the present invention, stabilizers, preservatives, excipients, and the like. The preferred lipids are the phospholipids and phosphatidyl cholines (lecithins), both natural and synthetic. Methods to form liposomes are known in the art. See, for example, Prescott, Ed., Methods in Cell Biology, Volume XIV, Academic Press, New York, N.W., p. 33 et seq. (1976).

While the compounds of the invention can be administered as the sole active pharmaceutical agent, they can also be used in combination with one or more other agents used in the treatment of cancer. The compounds of the present invention are also useful in combination with known therapeutic agents and anti-cancer agents, and combinations of the presently disclosed compounds with other anti-cancer or chemotherapeutic agents are within the scope of the invention. Examples of such agents can be found in Cancer Principles and Practice of Oncology, V. T. Devita and S. Hellman (editors), 6th edition (Feb. 15, 2001), Lippincott Williams & Wilkins Publishers. A person of ordinary skill in the art would be able to discern which combinations of agents would be useful based on the particular characteristics of the drugs and the cancer involved. Such anti-cancer agents include, but are not limited to, the following: estrogen receptor modulators, androgen receptor modulators, retinoid receptor modulators, cytotoxic/cytostatic agents, antiproliferative agents, prenyl-protein transferase inhibitors, HMG-CoA reductase inhibitors and other angiogenesis inhibitors, inhibitors of cell proliferation and survival signaling, apoptosis inducing agents and agents that interfere with cell cycle checkpoints. The compounds of the invention are also useful when co-administered with radiation therapy.

Therefore, in one embodiment of the invention, the compounds of the invention are also used in combination with known anticancer agents including, for example, estrogen receptor modulators, androgen receptor modulators, retinoid receptor modulators, cytotoxic agents, antiproliferative agents, prenyl-protein transferase inhibitors, HMG-CoA reductase inhibitors, HIV protease inhibitors, reverse transcriptase inhibitors, and other angiogenesis inhibitors.

In certain presently preferred embodiments of the invention, representative agents useful in combination with the compounds of the invention for the treatment of cancer include, for example, irinotecan, topotecan, gemcitabine, 5-fluorouracil, leucovorin carboplatin, cisplatin, taxanes, tezacitabine, cyclophosphamide, vinca alkaloids, imatinib (Gleevec), anthracyclines, rituximab, trastuzumab, as well as other cancer chemotherapeutic agents.

The above compounds to be employed in combination with the compounds of the invention will be used in therapeutic amounts as indicated in the Physicians' Desk Reference (PDR) 47th Edition (1993), or such therapeutically useful amounts as would be known to one of ordinary skill in the art.

The compounds of the invention and the other anticancer agents can be administered at the recommended maximum clinical dosage or at lower doses. Dosage levels of the active compounds in the compositions of the invention may be varied so as to obtain a desired therapeutic response depending on the route of administration, severity of the disease and the response of the patient. The combination can be administered as separate compositions or as a single dosage form containing both agents. When administered as a combination, the therapeutic agents can be formulated as separate compositions, which are given at the same time or different times, or the therapeutic agents, can be given as a single composition.

The present invention will be understood more readily by reference to the following examples, which are provided by way of illustration and are not intended to be limiting of the present invention.

Representative side chains for use in the compounds of the following examples may generally be prepared in accordance with the following procedures:

### EXAMPLES

Examples, methods and assays are described in pages 24 to 140 of published corresponding international application WO2009/109576, wherein Ex. No. 1-4, 6, 7, 10, 12-16, 18-21, 25-30 and 32-99 are reference examples and methods 7 and 8 are reference methods.

## Claims

1. A compound of Formula I, or a stereoisomer, tautomer, or pharmaceutically
acceptable salt thereof, 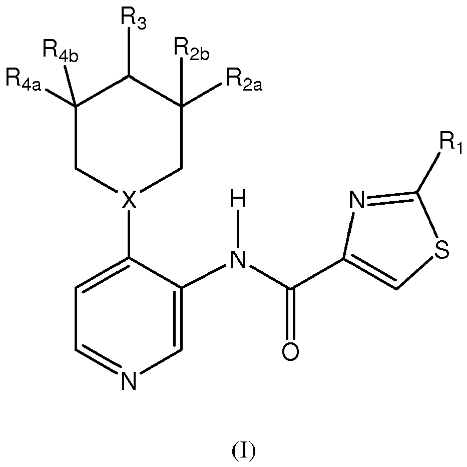 wherein,
R₁ is selected from 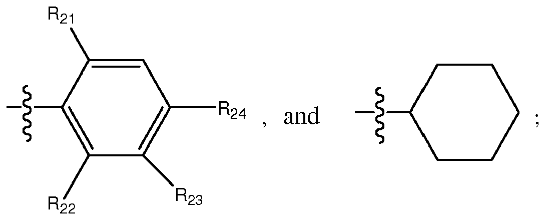
X represents CH, or N;
R₂ₐ is selected from amino, methyl, CH₂F, CF₃, C₂H₅, and H; R_{2b} is selected from H, and methyl;
R₃ is selected from H, OH, OCH₃, CH₃, F, and Cl;
R₄ₐ is selected from OH, OCH₃, OC₂H₅ and F;
R_{4b} is selected from methyl, H, and F;
R₂₁ represents H or F;
R₂₂ represents H, Cl, or F;
R₂₃ represents F, OC₂H₅, OCH₃, Cl, H, methyl, OH, or OCH(CH₃)₂; and
R₂₄ represents H or OH.

2. A compound of formula (I) according to Claim 1 selected from the group consisting of
| **Structure** | **Compound Name** |
|---|---|
| 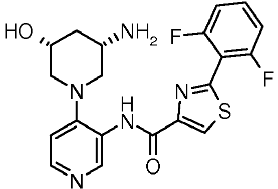 | N-(4-((3S,5R)-3-amino-5-hydroxypiperidin-1-yl) pyridin-3-yl)-2-(2,6-difluorophenyl)thiazole-4-carboxamide |
| 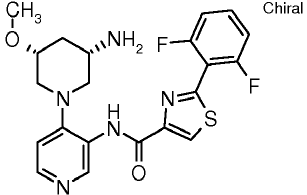 | N-(4-((3S,5R)-3-amino-5-methoxypiperidin-1-yl) pyridin-3-yl)-2-(2,6-difluorophenyl) thiazole-4-carboxamide |
| 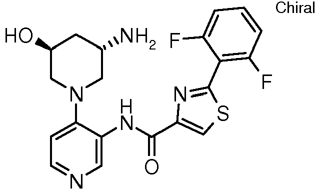 | N-(4-((3S,5S)-3-amino-5-hydroxypiperidin-1-yl) pyridin-3-yl)-2-(2,6-difluorophenyl)thiazole-4-carboxamide |
| 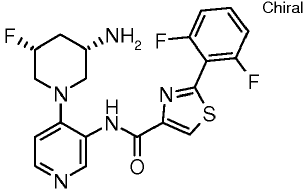 | N-(4-((3S,5R)-3-amino-5-fluoropiperidin-1-yl) pyridin-3-yl)-2-(2,6-difluorophenyl)thiazole-4-carboxamide |
| 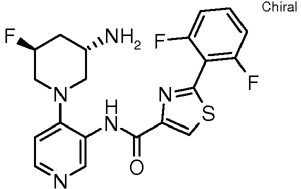 | N-(4-((3S,5S)-3-amino-5-fluoropiperidin-1-yl)pyridin-3-yl)-2-(2,6-difluorophenyl)thiazole-4-carboxamide |
| 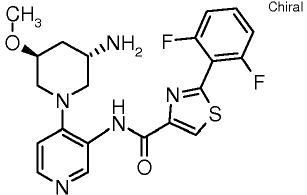 | N-(4-((3S,5S)-3-amino-5-methoxypiperidin-1-yl)pyridin-3-yl)-2-(2,6-difluorophenyl)thiazole-4-carboxamide |
| 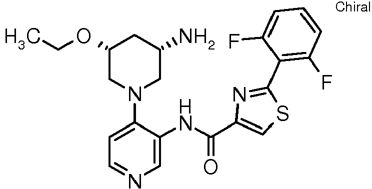 | N-(4-((3S,5R)-3-amino-5-ethoxypiperidin-1-yl)pyridin-3-yl)-2-(2,6-difluorophenyl)thiazole-4-carboxamide |
| 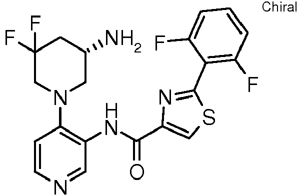 | (S)-N-(4-(5-amino-3,3-difluoropiperidin-1-yl)pyridin-3-yl)-2-(2,6-difluorophenyl)thiazole-4-carboxamide |
| 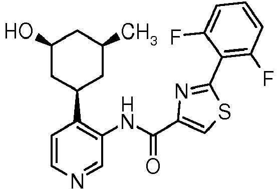 | 2-(2,6-difluorophenyl)-N-(4-((1R,3S,5S)-3-hydroxy-5-methylcyclohexyl)pyridin-3-yl)thiazole-4-carboxamide |

3. A compound according to Claim 1 or Claim 2 for use as a therapeutic agent.

4. A compound according to Claim 1 or Claim 2 for use in the treatment of cancer.

5. The compound for use according to claim 4, wherein the use is to treat cancer selected from the group consisting of lung carcinoma, pancreatic carcinoma, thyroid carcinoma, ovarian carcinoma, bladder carcinoma, breast carcinoma, prostate carcinoma, colon carcinoma, melanoma, myeloid leukemia, multiple myeloma, erythroleukemia, adenomas, and sarcomas.

6. A composition comprising a therapeutically effective amount of compound of according to Claim 1 or Claim 2, or a stereoisomer, tautomer, or pharmaceutically acceptable salt thereof, together with a pharmaceutically acceptable carrier.

7. A composition according to claim 6 for use in the treatment of cancer.

8. The composition for use according to claim 7, wherein the use is to treat cancer selected from the group consisting of lung carcinoma, pancreatic carcinoma, thyroid carcinoma, ovarian carcinoma, bladder carcinoma, breast carcinoma, prostate carcinoma, colon carcinoma, melanoma, myeloid leukemia, multiple myeloma, erythroleukemia, adenomas, and sarcomas.

9. A compound according to claims 1 or 2 for use as active pharmaceutical agent in combination with one or more other agents used in the treatment of cancer.

## Patentansprüche

1. Verbindung der Formel I oder ein Stereoisomer, Tautomer oder ein pharmazeutisch verträgliches Salz davon, 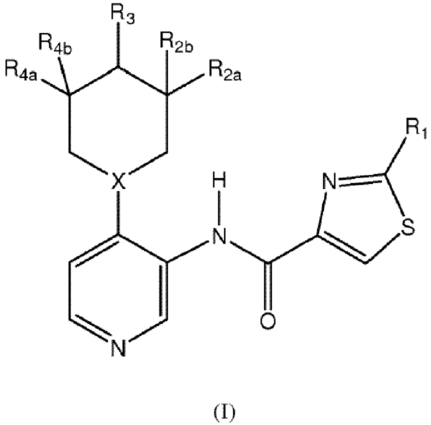 wobei,
R₁ ausgewählt ist aus 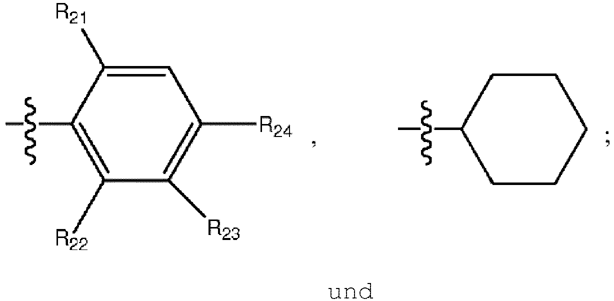
X CH oder N darstellt;
R₂ₐ ausgewählt ist aus Amino, Methyl, CH₂F, CF₃, C₂H₅ und H; R_{2b} ausgewählt ist aus H und Methyl;
R₃ ausgewählt ist aus H, OH, OCH₃, CH₃, F und Cl;
R₄ₐ ausgewählt ist aus OH, OCH₃, OC₂H₅ und F;
R_{4b} ausgewählt ist aus Methyl, H und F;
R₂₁ H oder F darstellt;
R₂₂ H, Cl oder F darstellt;
R₂₃ F, OC₂H₅, OCH₃, Cl, H, Methyl, OH oder OCH(CH₃)₂ darstellt; und
R₂₄ H oder OH darstellt.

2. Verbindung der Formel (I) nach Anspruch 1, ausgewählt aus der Gruppe bestehend aus
| **Struktur** | **Bezeichnung der Verbindung** |
|---|---|
| 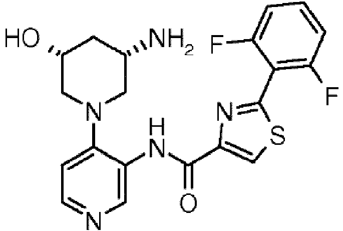 | N-(4-((3S,5R)-3-Amino-5-hydroxypiperidin-1-yl)pyridin-3-yl)-2-(2,6-difluorphenyl)thiazol-4-carboxamid |
| 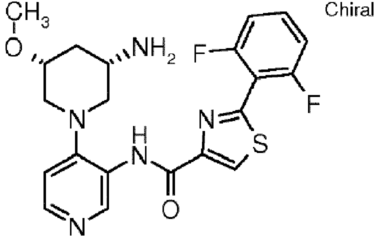 | N-(4-((3S,5R)-3-Amino-5-methoxypiperidin-1-yl)pyridin-3-yl)-2-(2,6-difluorphenyl)thiazol-4-carboxamid |
| 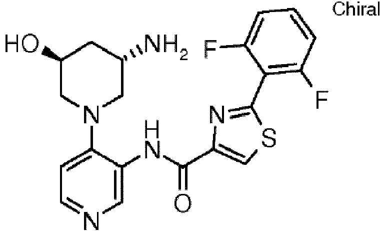 | N-(4-((3S,5S)-3-Amino-5-hydroxypiperidin-1-yl)pyridin-3-yl)-2-(2,6-difluorphenyl)thiazol-4-carboxamid |
| 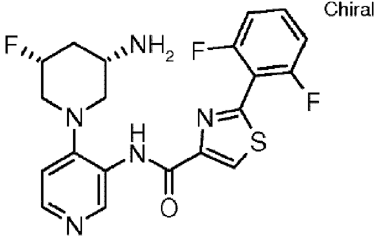 | N-(4-((3S,5R)-3-Amino-5-fluorpiperidin-1-yl)pyridin-3-yl)-2-(2,6-difluorphenyl)thiazol-4-carboxamid |
| 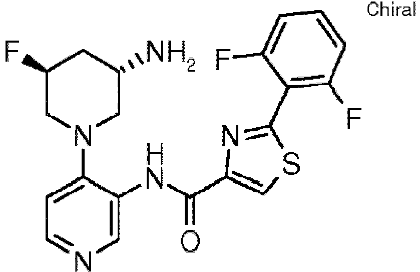 | N-(4-((3S,5S)-3-Amino-5-fluorpiperidin-1-yl)pyridin-3-yl)-2-(2,6-difluorphenyl)thiazol-4-carboxamid |
| 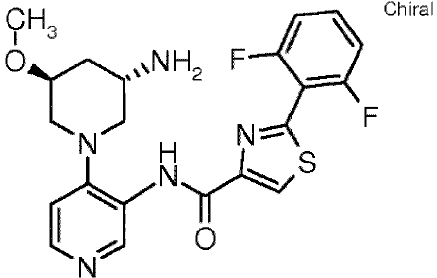 | N-(4-((3S,5S)-3-Amino-5-methoxypiperidin-1-yl)pyridin-3-yl)-2-(2,6-difluorphenyl)thiazol-4-carboxamid |
| 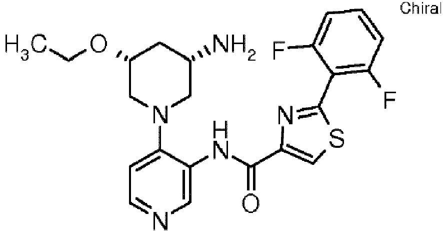 | N-(4-((3S,5R)-3-Amino-5-ethoxypiperidin-1-yl)pyridin-3-yl)-2-(2,6-difluorphenyl)thiazol-4-carboxamid |
| 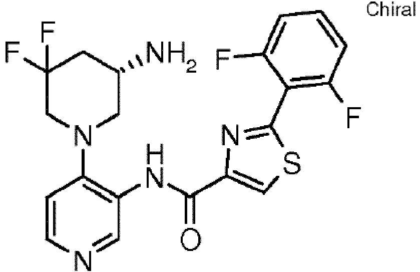 | (S)-N-(4-(5-Amino-3,3-difluorpiperidin-1-yl)pyridin-3-yl)-2-(2,6-difluorphenyl)thiazol-4-carboxamid |
| 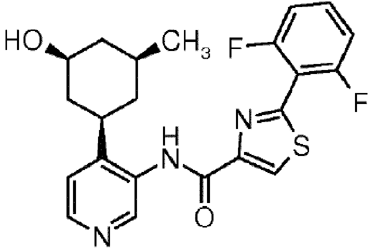 | 2-(2,6-Difluorphenyl)-N-(4-((1R,3S,5S)-3-hydroxy-5-methylcyclohexyl)pyridin-3-yl)thiazol-4-carboxamid |

3. Verbindung nach Anspruch 1 oder Anspruch 2 zur Verwendung als therapeutischer Wirkstoff.

4. Verbindung nach Anspruch 1 oder Anspruch 2 zur Verwendung in der Behandlung von Krebs.

5. Verbindung zur Verwendung nach Anspruch 4, wobei die Verwendung darin besteht, Krebs aus der Gruppe bestehend aus Lungenkarzinom, Pankreaskarzinom, Schilddrüsenkarzinom, Ovarialkarzinom, Blasenkarzinom, Brustkarzinom, Prostatakarzinom, Dickdarmkarzinom, Melanom, myeloischer Leukämie, multiplem Myelom, Erythrämie, Adenomen und Sarkomen zu behandeln.

6. Zusammensetzung, die eine therapeutisch wirksame Menge einer Verbindung nach Anspruch 1 oder Anspruch 2 oder ein Stereoisomer, Tautomer oder pharmazeutisch verträgliches Salz davon umfasst, zusammen mit einem pharmazeutisch verträglichen Träger.

7. Zusammensetzung nach Anspruch 6 zur Verwendung in der Behandlung von Krebs.

8. Zusammensetzung zur Verwendung nach Anspruch 7, wobei die Verwendung darin besteht, Krebs aus der Gruppe bestehend aus Lungenkarzinom, Pankreaskarzinom, Schilddrüsenkarzinom, Ovarialkarzinom, Blasenkarzinom, Brustkarzinom, Prostatakarzinom, Dickdarmkarzinom, Melanom, myeloischer Leukämie, multiplem Myelom, Erythrämie, Adenomen und Sarkomen zu behandeln.

9. Verbindung nach Anspruch 1 oder 2 zur Verwendung als pharmazeutischer Wirkstoff in Kombination mit einem oder mehreren anderen Wirkstoffen, die in der Behandlung von Krebs verwendet werden.

## Revendications

1. Composé de formule I, ou stéréo-isomère, tautomère,
ou sel pharmaceutiquement acceptable de celui-ci 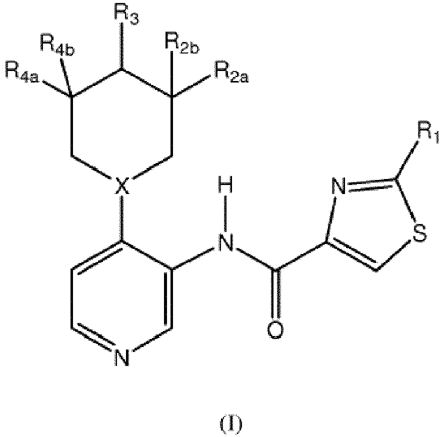 dans lequel
R₁ est sélectionné parmi 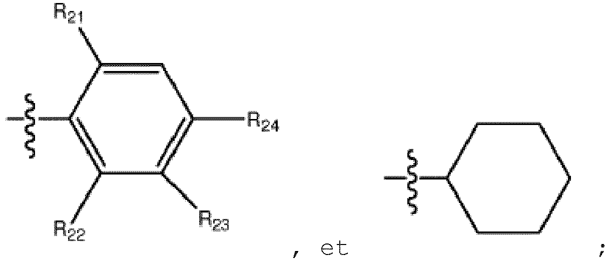
X représente CH, ou N ;
R₂ₐ est choisi parmi amino, méthyle, CH₂F, CF₃, C₂H₅, et H ;
R_{2b} est choisi parmi H et méthyle ;
R₃ est choisi parmi H, OH, OCH₃, CH₃, F et Cl ;
R₄ₐ est choisi parmi OH, OCH₃, OC₂H₅ et F ;
R_{4b} est choisi parmi méthyle, H et F ;
R₂₁ représente H ou F ;
R₂₂ représente H, Cl ou F ;
R₂₃ représente F, OC₂H₅, OCH₃, Cl, H, méthyle, OH ou OCH(CH₃)₂ ; et
R₂₄ représente H ou OH.

2. Composé de formule (I) selon la revendication 1 choisi dans le groupe comportant
| **Structure** | **Nom du composé** |
|---|---|
| 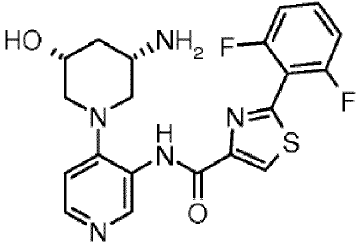 | N-(4-((3S,5R)-3-amino-5-hydroxypipéridin-1-yl)pyridin-3-yl)-2-(2,6-difluorophényl)thiazole-4-carboxamide |
| 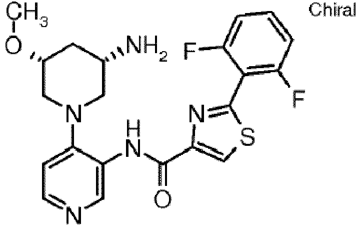 | N-(4-((3S,5R)-3-amino-5-methoxypipéridin-1-yl) pyridin-3-yl)-2-(2,6-difluorophényl) thiazole-4-carboxamide |
| 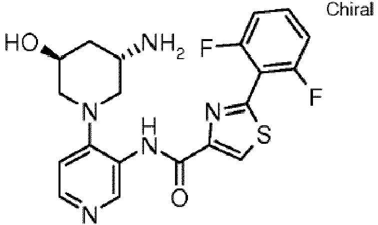 | N-(4-((3S,5S)-3-amino-5-hydroxypipéridin-1-yl) pyridin-3-yl)-2-(2,6-difluorophényl)thiazole-4-carboxamide |
| 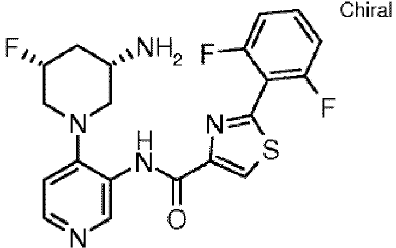 | N-(4-((3S,5R)-3-amino-5-fluoropipéridin-1-yl) pyridin-3-yl)-2-(2,6-difluorophenyl)thiazole-4-carboxamide |
| 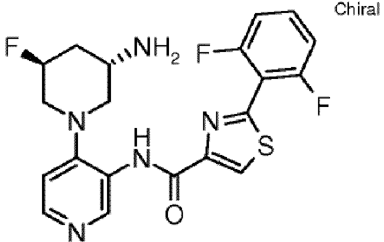 | N-(4-((3S,5S)-3-amino-5-fluoropipéridin-1-yl)pyridin-3-yl)-2-(2,6-difluorophényl)thiazole-4-carboxamide |
| 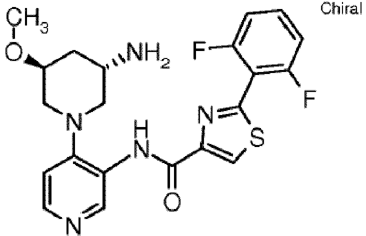 | N-(4-((3S,5S)-3-amino-5-methoxypipéridin-1-yl)pyridin-3-yl)-2-(2,6-difluorophényl)thiazole-4-carboxamide |
| 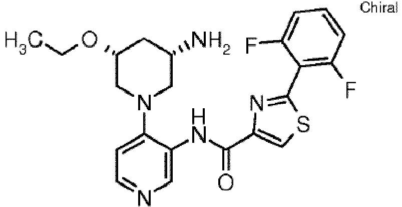 | N-(4-((3S,5R)-3-amino-5-éthoxypipéridin-1-yl)pyridin-3-yl)-2-(2,6-difluorophényl)thiazole-4-carboxamide |
| 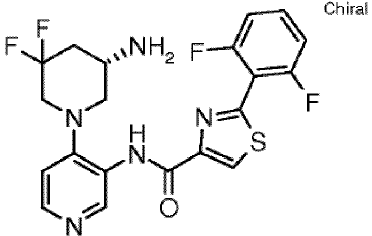 | (S)-N-(4-(5-amino-3,3-difluoropipéridin-1-yl)pyridin-3-yl)-2-(2,6-difluorophényl)thiazole-4-carboxamide |
| 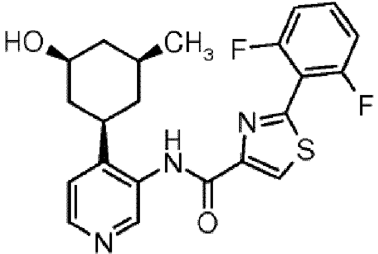 | 2-(2,6-difluorophényl)-N-(4-((1R,3S,5S)-3-hydroxy-5-méthylcyclohexyl)pyridin-3-yl)thiazole-4-carboxamide |

3. Composé selon la revendication 1 ou la revendication 2 destiné à être utilisé comme agent thérapeutique.

4. Composé selon la revendication 1 ou la revendication 2 destiné à être utilisé dans le traitement du cancer.

5. Composé destiné à être utilisé selon la revendication 4, dans lequel l'utilisation consiste à traiter un cancer choisi dans le groupe comprenant le carcinome du poumon, le carcinome pancréatique, le carcinome de la thyroïde, le carcinome ovarien, le carcinome de la vessie, le carcinome du sein, le carcinome de la prostate, le carcinome du côlon, le mélanome, la leucémie myéloïde, le myélome multiple, l'érythroleucémie, les adénomes et les sarcomes.

6. Composition comprenant une quantité thérapeutiquement efficace d'un composé selon l'une des revendications 1 ou 2, ou un stéréo-isomère, un tautomère, ou un sel pharmaceutique acceptable de celle-ci, conjointement avec un support pharmaceutiquement acceptable.

7. Composition selon la revendication 6 destinée à être utilisée dans le traitement du cancer.

8. Composition destiné à être utilisée selon la revendication 7, dans laquelle l'utilisation consiste à traiter un cancer choisi dans le groupe comprenant le carcinome du poumon, le carcinome pancréatique, le carcinome de la thyroïde, le carcinome ovarien, le carcinome de la vessie, le carcinome du sein, le carcinome de la prostate, le carcinome du côlon, le mélanome, la leucémie myéloïde, le myélome multiple, l'érythroleucémie, les adénomes et les sarcomes.

9. Composé selon la revendication 1 ou 2 destiné à être utilisé comme agent pharmaceutique actif en combinaison avec un ou plusieurs autres agents utilisés dans le traitement du cancer.
